# FASCICULE DE BREVET EUROPEEN

(11) **EP 2 411 390 B1**
(45) Date de publication et mention de la délivrance du brevet: **29.01.2014**
(21) Numéro de dépôt: 10711212.0
(22) Date de dépôt: 25.03.2010
(51) Int. Cl.: G01R 33/28, G01R 33/56

(54) **COMPOSES BIRADICALAIRES DE TYPE DINITROXYDE OPTIMISES POUR LA POLARISATION DYNAMIQUE NUCLEAIRE (PDN)**
FÜR DIE DYNAMISCHE KERNPOLARISIERUNG (DYNAMIC NUCLEAR POLARISATION, DNP) OPTIMIERTE DINITROXIDBIRADIKALVERBINDUNGEN
DINITROXIDE BIRADICAL COMPOUNDS OPTIMIZED FOR DYNAMIC NUCLEAR POLARIZATION (DNP)

(30) Priorité: 26.03.2009 FR 0901454
(43) Date de publication de la demande: 01.02.2012
(73) Titulaire: Universite d'Aix Marseille, 13284 Marseille Cedex 07 (FR); Massachusetts Institute of Technology, Cambridge, Massachusetts 02139 (US)
(72) Inventeur: OUARI, Olivier, F-74600 Seynod (FR); KAROUI, Hakim, F-13001 Marseille (FR); LE MOIGNE, François, F-13012 Marseille (FR); TORDO, Paul, F-13010 Marseille (FR); GRIFFIN, Robert G., Newton Massachusetts 02460 (US); MATSUKI, Yoh, Osaka 5650871 (JP); MALY, Thorsten, Cambridge Massachusetts 02139 (US)
(74) Mandataire: Tezier Herman, Béatrice
(86) Numéro de dépôt international: PCT/EP2010/053954
(87) Numéro de publication internationale: WO 2010/108995

(56) Documents cités:
- WO-A1-02/48205
- WO-A2-2005/024440
- FR-A- 1 526 656
- FR-A1- 2 123 501
- JP-A- 4 362 632
- US-A- 4 921 962
- US-A- 5 204 473
- CHIARELLI R ET AL: "Synthèse du dupeyredioxyle (1,3,5,7-tétraméthyl-2,6-diazaadamantane-2 ,6-dioxyle), biradical nitroxyde à propriétés ferromagnétiques" BULLETIN DE LA SOCIETE CHIMIQUE DE FRANCE, SOCIETE FRANCAISE DE CHIMIE. PARIS, FRANCE, vol. 130, no. 3, 1 janvier 1993 (1993-01-01), pages 299-303, XP008120718 ISSN: 0037-8968
- RASSAT, ANDRE: "Magnetic properties of nitroxide multiradicals" PURE AND APPLIED CHEMISTRY, vol. 62, no. 2, 1990, pages 223-227, XP002576221
- HUANG, WENLI ET AL: "Unique Behavior of Nitroxide Biradicals in the Controlled Radical Polymerization of Styrene" MACROMOLECULES, vol. 35, no. 6, 2002, pages 2305-2317, XP002575991
- TODA, TOSHIMASA ET AL: "Stable free radicals. XI. ESR Spectra of Stable Nitroxide Biradicals" BULLETIN OF THE CHEMICAL SOCIETY OF JAPAN, vol. 45, no. 6, 1972, pages 1764-1766, XP002575992
- TODA, TOSHIMASA ET AL: "Stable free radicals. VI. Synthesis of substituted 4-imidazolidinone-1-oxyls" BULLETIN OF THE CHEMICAL SOCIETY OF JAPAN, vol. 44, no. 12, 1971, pages 3445-3450, XP002575993
- GAGNAIRE, GENEVIEVE ET AL: "Regulation by potassium ions of spin exchange and dipolar splitting in a biradical. A simple allosteric system" TETRAHEDRON LETTERS, vol. 30, no. 47, 1989, pages 6507-6510, XP3001849
- DUPEYRE, ROSE M. ET AL: "Nitroxides. LII. Synthesis and electron spin resonance studies of N,N'-dioxy-2,6-diazaadamantane, a symmetrical ground state triplet" JOURNAL OF THE AMERICAN CHEMICAL SOCIETY, vol. 96, no. 21, 1974, pages 6559-6568, XP002575995
- FLUEKIGER, P. ET AL: "Chirality and spin density: ab initio and density-functional approaches" INTERNATIONAL JOURNAL OF QUANTUM CHEMISTRY, vol. 45, no. 6, 1993, pages 649-663, XP002575996
- MICHON, J. ET AL: "Nitroxides. 88. ESR conformational study of a biradical" TETRAHEDRON, vol. 36, no. 7, 1980, pages 871-876, XP002575997
- MILLER, JOEL S. ET AL: "Organic and organometallic molecular magnetic materials: designer magnets" ANGEW. CHEM., INT. ED. ENGL., vol. 33, no. 4, 1994, pages 385-415, XP002575998
- HU, KAN-NIAN ET AL: "Dynamic Nuclear Polarization with Biradicals" JOURNAL OF THE AMERICAN CHEMICAL SOCIETY, vol. 126, no. 35, 2004, pages 10844-10845, XP002575999 cité dans la demande
- SONG, CHANGSIK ET AL: "TOTAPOL: A Biradical Polarizing Agent for Dynamic Nuclear Polarization Experiments in Aqueous Media" JOURNAL OF THE AMERICAN CHEMICAL SOCIETY, vol. 128, no. 35, 2006, pages 11385-11390, XP002576000
- JOO, CHAN-GYU ET AL: "In Situ Temperature Jump High-Frequency Dynamic Nuclear Polarization Experiments: Enhanced Sensitivity in Liquid-State NMR Spectroscopy" JOURNAL OF THE AMERICAN CHEMICAL SOCIETY, vol. 128, no. 29, 2006, pages 9428-9432, XP002494329
- HU, KAN-NIAN ET AL: "High-frequency dynamic nuclear polarization using biradicals: A multifrequency EPR lineshape analysis" JOURNAL OF CHEMICAL PHYSICS, vol. 128, no. 5, 2008, pages 052302/1-052302/18, XP009115599

## Description

La présente invention relève du domaine de la chimie organique et en particulier celui des radicaux libres organiques utilisés comme agents de polarisation dans la technique de Polarisation Dynamique Nucléaire (PDN), qui met en jeu le transfert d'une polarisation de spins électroniques vers des noyaux d'un composé dont on observe le spectre de Résonance Magnétique Nucléaire (RMN). Elle concerne des agents de polarisation ayant une structure moléculaire particuliére permettant d'obtenir à base température et à haut champ un transfért de polarisation optimal et une augmentation optimale des signaux de RMN/MAS des noyaux polarisés du composé étudié. Lorsque l'èchantillon solide étudié a été polarisé, il peut être dissous et si la relaxation nucléaire n'est pas trop rapide, on obtient une soution «hyperpolarisée» de l'échantillon, qui peut être utilisée pour des observations de RMN en phase liquide on d'Imagerie pas Résonance Magnétique Nucléaire (IRM).

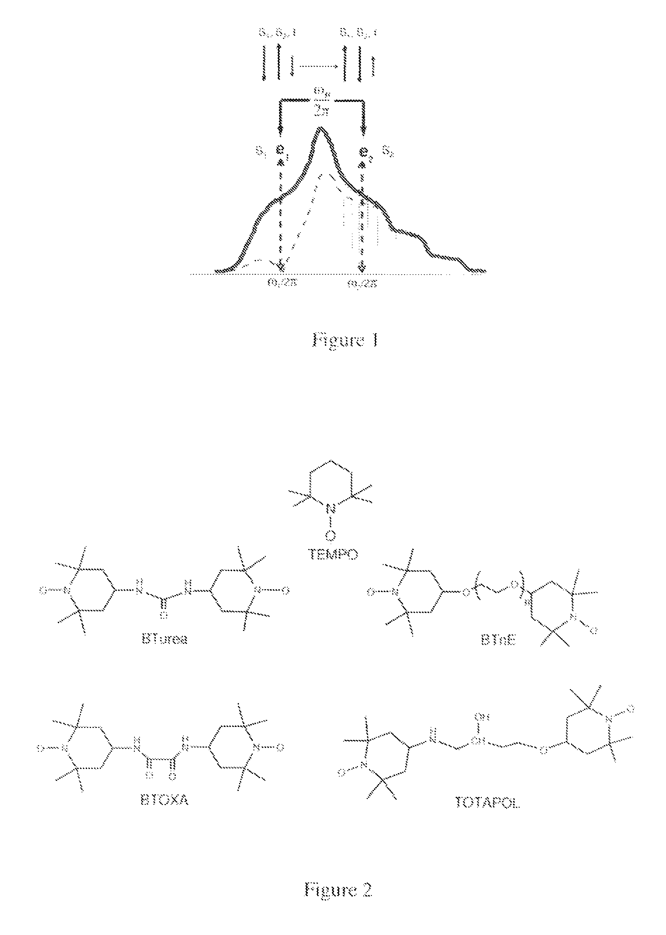

Plusieurs mécanismes sont impliqués dans la PDN à basse température, parmi eux le «Cross Effect » (CE) et le «Thermal Mixing » sont ceux qui permettant d'obtenir la polarisation maximale et donc l'accroisement maximal des signaux RMN Ces deux mécanismes reposent essentiellement sur un processus à trois spins deux spine électroniques S₁ et S₂ couplés à travers une interation dipôle/dipôle et le spin 1 du noyau a que l'on cherche à polariser (Figure 1). Si on pompe l'échantillon à la fréquence ω₁/2π: et si la fréquence ω₂/2π est telle que la différence (ω₂/2π) - ω₁/2π) est égale à la fréquence de Larmor (ω₃/2π) des noyaux n, un mécanisme flip-flop impliquant les trois spins et conservant l'énergie conduira à la polarisation de ces noyaux.

Les nitroxydes stables comme le TEMPO sont des agents de polarisation trés utilisés en PDN en phase solide à haut champ. Avec des concentrations de l'ordre de 40 mM de TEMPO, des facteurs d'augmentation de la polarisation, a, de l'ordre de 40 ont été observés pour le noyau de ¹³C de l'urée. Si on baisse la concentration, le facteur d'augmentation chute rapidement car l'interaction dipôle S₁/dipôle S₂ devient moins efficace. R.G. Griffin a montré (R. G. Griffin et al.: J. Am. Chem. Soc. 126, 10844-10845. 2004) que si on introduit les deux spins S₁ et S₂ dans la même molécule, la polarisation nucléaire obtenue est plus efficace et ce, pour des concentrations plus faibles en agent polarisant. Il a utilisé divers dinitroxydes (Figure 2) formés par deux unités TEMPO réunies par différents types de bras espacées, tels que bis-TEMPO-n-éthyléne glycol (BTnE) (n indiquant le nombre d'unités éthylène glycol), bis-TEMPO-Urée (BTurea), bis-TEMPO-oxalamide (BTOXA) et en particulier 1-(TEMPO-4-oxy)-3-(TEMPO-4-amino)propan-2-ol (TOTAPOL).

Les facteurs d'accroissemnt de la polarisation observés (à 90 K, pour un champ de 5 T et une puissance d'irradiation de 1,5 W) dans le cas du noyau de ¹³C de l'urée (R.G. Griffin et al ; J. Am. Chem. Soc., 128, 11385-11390, 2006 _{;} J. Chem. Phy. 128, 052302-1052302-17, 2008) vont de ε = 175 ≠ 20 avec le BT2B (5 mM) ou a = 190 ± 20 avec le TOTAPOL (5mM) alors que ε = 40 ± 5 seulement avec le TEMPO (40 mM).

En théorie, pour ces dinitroxydes, dans les conditions de travail utilisées, l'égalité ω₂/2π - ω₁/2π = ωₐ/2π sera pratiquement vérifiée pour les molécules adoptant une conformation dans laquelle les plans nodaux des systémes a des deux groupes aminoxyde font entre eux un angle proche de 90°. Ceci est une conséquence de la forte anisotrope du facteur de Landé g du TEMPO (gₓₓ =: 2,0090 ; g_{yy} = 2,0061; g_{zz} = 2,0021). Toutefois, dans ces molécules il n'existe pss de contraintes structurales particuliéres permettant de bolquer la géométrie moléculaire de façon à ce que les plans nodaux des deux groupes aminoxyle des deux unités nitroxyle fassent entre eux un angle proche de 90°. Ainsi, la flexibilité de la jonction entre les deux unités nitroxyde de ces diffèrents nitroxyde n'est pas propice à l'obtention d'une polarisation optimale. L'objet de la présente invention est de fournir des composés de type dinitroxyde présentant une jonction particuliére entre les deux cycles dont les caractéristiques assurent un transfert de polarisation optimal constant en PDN. La présente invention répond ainsi au besoin d'agents de polarisation permettant d'obtenir un transfert de polarisation optimal en PDN afin d'augmenter les performances de la RMN en phase solide ou liquide et de l'IRM.

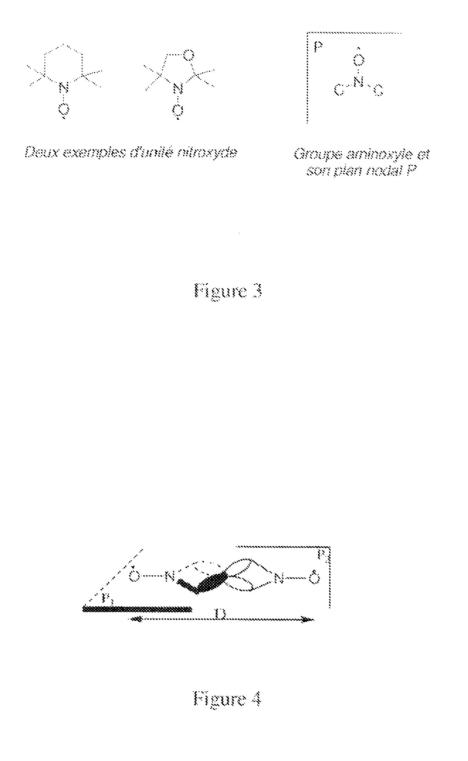

Dans la description de la présente invention les ternes « unité(s) nitroxyde » et « groupe aminoxyle » se rèférent aux entités décrites dans la Figure 3 qui représente deux exemples d'unités nitroxyde et un groupe aminoxyle dans son plan nodal P.

La présente invention concerne des agents de polarisation de type dinitroxyde dans lesquels les deux groupes aminoxyle appartiennent à des cycles, de plus, la jonction entre les deux groupes aminoxyle constitue une jonction qui permet :
- de fixer la distance entre les deux groupe aminoxyle, ce qui permet de contrôler l'interaction dipôle-dipôle.
- de maintenir l'angle que font entre eux les plans nodaux des deux groupes aminoxyle à une valeur proche de 90° (Figure 4).

Selon un premier objet, la présente invention concerne des composés biradicalaire de type dinitroxyde de formule générale : 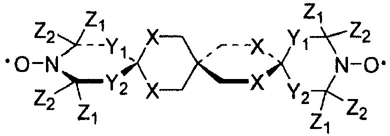 dans laquelle X est une simple liaison, ou O, N, N(O), S, S(O), SO₂, ou une chaîne (C1-C4) alkyle,
dans lesquelles Y1 est une simple liaison, ou O, N, N(O), S, S(O), SO₂, ou une chaîne (C1-C4) alkyle,
dans lesquelles Y2 est une simple liaison, ou O, N, N(O), S, S(O), SO₂, ou une chaîne (C1-C4) alkyle,
dans lesquelles Z₁ ou Z₂ = -R₁, et R2 est un éventuel substituant de R1, avec R₁ étant une chaîne (C1-C17) alkyle, (C1-C17) alkényle, (C1-C17) alkynyle, (C1-C17) cycloalkyle, (C1-C17) hétérocycloalkyle, (C6-C 18) aryle et R₂ = H ou est choisi parmi un hydroxyle (-OH), amine primaire (-NH₂), amine secondaire(-NH), tertiaire(-N), ammonio (-N⁺), oxyde d'amine (-NO), carboxyle (-COOH), sulfanyle(-SH), sulfinyle (-SO), sulfonyle (-SO₂), sulfonato (-SO₃⁻), phosphono (-PO₃H₂, -PO₃(R)₂ où R= (C1-C17) alkyle, (C6-C18) aryle), phosphoryle (-P(O)(R)₂ où R= (C1-C17) alkyle, (C6-C18) aryle), phosphonio (-P⁺), ester phosphorique (-O-PO₃), et Z1 et Z2 combinés ensemble avec le même atome de carbone cyclique auquel ils sont liés peuvent former un (C1-C17) spirocycloalkyle ou un (C1-C17) spirohétérocycloalkyle éventuellement substitué par R2.

En outre, les composés de type dinitroxyde de la présente invention sont caractérisés en ce que la jonction reliant les unités nitroxyde, permet l'obtention d'une conformation stable qui maintient l'angle que font entre eux les plans nodaux des groupes aminoxyle des deux unités P₁ et P₂ (Figure 4), dans une plage comprise entre 65° et 115°.

De plus, les composés biradicalaires de type dinitroxyde de la prèsente invention sont caractérisés en ce que leur jonction permet le maintien des atomes d'azote des groupes aminoxyle, compris dans les unités nitroxyde, à une distance comprise entre 3 Ä et 16 Ä.

De manière préférée, les unités nitroxyde constituant les composés biradicalaires dinitroxydes de la présente invention sont sélectionnées parmi des unités pipéridinoxyle, pyrrolidinoxyle, imidazolinoxyle, imidazolidinoxyle, oxazolidinoxyle et nitronylatroxide.

Dans un autre mode préféré de l'invention, les composés biradicalaires de type nitroxyde de la présente invention sont caractérisés par l'adjonction d'au moins un groupement chimique. Dans un mode particulier de ce mode préféré, le/les groupements chimiques ajoutés aux composés biradicalaires de la présente invention facilitent la solubilité dans l'eau desdits composés biradicalaires et sont sélectionnés parmi le groupe constitué, à titre d'exemple, des fonctions et groupements suivants : hydroxyle ( -OH), amine primaire (-NH₂), amine secondaire (-NH), tertiaire (-N), ammonio (-N⁺), oxyde d'amine (-NO), carboxyle (-COOH), sulfanyle(-SH) sulfinyle (-SO), sulfonyle (-SO₂), solfonato (-SO₃). phosphono (-PO₃H₂, -PO₃R₃ où R= (C1-C17) alkyle, (C6-C18) aryle), phosphoryle (. P(O)R₂ où R= (C1-C17) alkyle (C6-C18) aryle), phosphonio (-P⁺), ester phosphorique P(O)R où R= (C1-C17) alkyle (C6/C18) aryle), (-P⁺), ester phosphorique (-O-PO₃⁻). Dans un autre mode particulier de ce mode préféré de l'invention, les composés biradicalaires de la présente invention sont caractérisés en ce qu'ils contiennent au moins un photosensibilisateur sélectionné parmi, par example, la thioxanthone, la benzoïne, le benzil déméthylcétal ou la benzophénone. Dans un autre mode particulier de l'invention, le/les groupements chimiques ajoutés aux composés biradicalaires de la présente invention sont réactifs vis à vis des groupements caractéristiques : -SH, -NH₂, -OH, -COOH, hydroxyaryle (ArOH) et permettent la conjugaison desdits composés biradicalaires à des molécules d'intérêt. Ces groupements chimiques sont des esters activés présentant les formules suivantes données à titre d'exemples non exhaustifs :

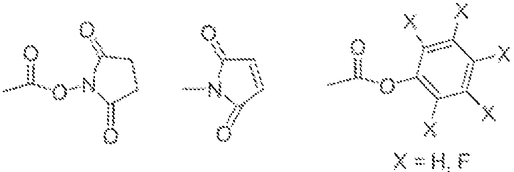

Dans un dernier mode particulier de ce mode préféré de l'invention, les composés biradicalaires de type dinitroxyde selon la présente invention sont caractérisés en ce qu'ils sont conjugués à des biomolécules telles que protéines, ribosomes, lipides, saccharides, ADN, ARN.

Dans un autre mode préféré de l'invention, les composés dinitroxyde selon la présente invention sont caractérisés par un marquage isotopique. Dans un mode de réalisation préféré, les composés dinitroxyde sont marqués isotopiquement par le Deutérium (²H). Dans un autre mode de réalisation préféré, les composés dinitroxyde sont marqués isotopiquement par l'Azote 15 (¹⁵N). Dans un autre mode de réalisation préféré, les composés dinitroxyde sont marqués isotopiquement par le Carbon 13 (¹³C).

Dans un dernier mode préféré de l'invention, les composés de la présente invention sont des composés paramagnétiques qui comprennent au moins un motif dinitroxyde composé des deux unités nitroxyde reliées par une jonction rigide telle que revendiquée dans la présente invention.

Selon un second objet, la présente invention concerne également une composition comprenant un moins un des composés dinitroxyde mentionnés dans les modes de réalisation précédentes.

Selon un troisième objet, la présente invention concerne également l'utilisation d'au moins un des composés dinitroxyde détaillés dans les modes de réalisation précédents, comme agent de polarisation. Dans un mode préféré, l'invention concerne l'utilisation d'au moins un des composés dinitroxyde de la présente invention comme agent de polarisation dans les techniques de biologie structurale, de Résonance Magnétique Nucléaire du solide ou appliquées à des échantillons liquides, dans les techniques de physique des particles ainsi que dans les techniques d'imagerie médicale. Dans un mode encore plus préféré, l'invention concerne l'utilisation d'au moins un des composés dinitroxyde de la présente invention comme agent de polarisation dans la technique de PDN.

### Bréve description des figures :

Figure 1 : Spectre de RPE du radical TEMPO en phase solide et illustration d'un processus flip-flop à trois spins, avec conservation de l'énergie conduisant à la polarisation des moyens n de spin 1.
Figure 2 : TEMPO et dinitroxydes utilisés par R. G. Griffin comme agents de polarisation, dans des expériences de PDN en phase solide à basse température (T ≤ 90 K).
Figure 3: Deux exemples d'unités nitroxyde et d'un groupe aminoxyle dans son plan nodal P.
Figure 4 : Disposition des plans nodaux des groupes aminoxyle dans les composés de l'invention.
Figure 5: Structure du bTbK déterminée par diffraction des rayons X.
Figure 6 : Courbe de croissance de la polarisation des ¹H du milieu en présence de bTbK, enregistrée pour la valeur du champ magnétique correspondant à une polarisation positive (DNP(+)). La polarisation des ¹H est détectée indirectement à travers le du ¹³C de l'urée par une séquence de polarisation croisée. Dans les encadrés sont représentés les spectres avec au sens irradiation, pour les valeurs du champ correspondant à DNP (+) et DNP(-).
Figure 7. Variation en fonction de la puissance d'irradiation, de l'accroisement de la polarisation maximale stationnaire (c⁺) pour le bTbK et le TOTAPOL enregistrée dans les mêmes conditions expérimentales.

Par unité nitroxyde, on entend un cycle à plusieurs atomes comprenant un groupe aminoxyle.

Par composés biradicalaires de type dinitroxyle, ou composés dinitroxyde on entend des composés comportant deux groupes aminoxyle respectivement compris dans deux unités nitroxyde distinctes telles que précédemment définies.

De manière préférée, les unités nitroxyde constituant les composés biradicalaires dinitroxyde de la présente invention sont sélectionnées parmi des unités pipéridinoxyle, pyrrolidinoxyle, imidazolinoxyle, imidazolidinoxyle, oxazolidinoxyle et nitronylnitroxide. Les composés biradicalaires de type dinitroxyde de la présente invention peuvent être constituée d'unités nitroxyde homologues telles que deux unités pipéridinoxyle, deux unités pyrrolidinoxyle, deux unités imidazolinoxyle, deux unités oxazolidinoxyle, deux unités imidazolidinoxyle ou deux unités nitronylnitroxide. Par exemple, les composés biradicalaires de la présente invention peuvent être constituées de deux unités tétraméthylpipéridinoxyle telles que dans le composé suivant :

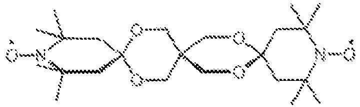

Les composés biradicalaires de type nitroxyde de la présente invention peuvent être constitués d'unités nitroxydes hétérologues sélectionnées parmi les unités nitroxydes mentionnées ci-dessus. Par exemple, les composés biradicalaires de la présente invention peuvent être constituées d'une part d'une unité tétraméthylpipéridinoxyle et d'autre part d'une unité oxazolidinoxyle.

Les composés biradicalaires de type dinitroxyde de la présente invention peuvent constituer un motif inclus dans un composé polynitroxyde comprenant trois, quatre, cinq, dix, ou n unités nitroxyde.

Les composés biradicalaires de type dinitroxyde de la présente invention sont caractérisés par une jonction rigide reliant les cycles comprenant les groupes aminoxyle. La jonction rigide dans les composés biradicalaires de type dinitroxyde entre les deux groupes aminoxyle permet le blocage des cycles contenant ces groupes, dans une conformation qui maintient l'angle que font entre eux leurs plans nodaux P₁ et P₂ (Figure 4), dans une plage comprise entre 68° et 115°. Cette jonction permet également le maintien des atomes d'azote des deux groupes aminoxyde a une distance comprise entre 3 Å et 16 Å ce qui permet de contrôler l'interaction dipôle-dipôle. Le contrôle de ces deux paramètres dans les composés dinitroxyde de la présente invention permet d'obtenir des conditions optimales pour le transfert de polarisation.

La jonction rigide entre les deux groupes aminoxyle selon la presente invention comporte un nombre impair de liaisons spiramiques.

Par liaison spiranique, on entend une liaison caractérisée par la présence d'un atome inclus dans deux cycles ou groupements cycliques non accolés. Une illustration de ce type de liaison est donnée ci-dessous.

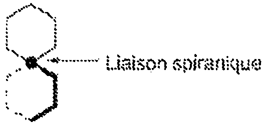

Ce type de liaison permet de lier les cycles contenant les unités nitroxyde dans les composés biradicalaires de la présente invention. Ce type de liaison permet de former une jonction rigide entre les cycles desdites unités et de bloquer celles-ci dans une orientation définie et favorable à un transfert de polarisation optimal. Par jonction rigide caractérisant les composés de la présente invention, on entend également un nombre impair de liaisons spiraniques successives permettant de maintenir les atomes d'azote des unités nitroxyde à une distance permettant un transfert de polarisation optimal. De manière préférée, la jonction rigide constituée par une ou plusieurs liaisons spiraniques permet de maintenir les atomes d'azote des unités nitroxyde à une distance comprise entre 3 Å et 16 Å.

Dans un mode préféré de la présente invention, la jonction rigide est constituée par une, trois ou cinq liaisons spiraniques successives. De manière encore plus préfèrée, la jonction rigide est constituée par une ou trois jonctions spiramiques successive. Par un bras comportant un nombre impair de liaisons spiraniques, on entend la jonction rigide qui permet à la fois de maintenir les unités nitroxyde des composés biradicalaires de la prèsente invention dans une conformation donnée et à une distance donnée. Dans ce même mode de réalisation préféré, la jonction rigide constituée par une ou plusieurs liaisons spiraniques permet de fixer les plans nodaux des deux groupes aminoxyle des composés biradicalaires de type dinitroxyde de la présente invention, relativement l'un par rapport à l'autre, dans un angle compris entre 65° et 115°. Préférentiellement, cet angle peut être compris entre 75° et 105°, ou 85° et 95°. De manière encore plus préfèrentielle, les plans nodaux des deux groupes aminoxyle des composés biradicalaires de type dinitroxyde de la prèsente invention sont orthogonaux.

Une illustration d'une jonction rigide constituée par trois liaisons spiraniques est donnée dans la formule ci-dessus ;

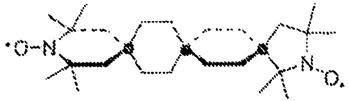

La présente invention propose un type de jonction rigides à servir un nombre impair de liaisons spiraniques permettant d'obtenir des composés biradicalaires de type dinitroxyde, constitués par des cycles contenant les deux unités nitroxyde dont les plans nodaux sont maintenues dans une orientation fixe et favorable à un transfert optimal de polarisation. Préférentiellement, ces troix types de jonctions rigides permettent de fixer les plans nodaux des deux groupes aminoxyle des unités nitroxyde relativement l'un par rapport à l'autre, dans un angle compris entre 65° et 115°, préférentiellement entre 75° et 105°, ou entre 85° et 95°. D'une manière autrement préférée, les plans des deux unités nitroxyde des composés de la présente invention sont orthogonaux. Ce type de jonction rigide permet ègalement de maintenir les atomes d'azote des deux unités nitroxyde, à une distance permettant un transfert de polarisation optimal. Préférentiellement, ces trois types de jonction permettent de maintenir les atomes d'azote des deux unités nitroxyde à une distance comprise entre 3 Å et 16 Å.

Dans un mode de réalisation préféré, un composé selon la présente invention possède la dénomination bis-TEMPO-bis-Ketal (bTbK) et présente la formule suivante :

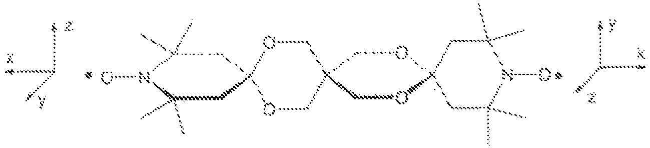

Dans un autre mode de réalisation la présente invention concerne des composés biradicalaires de type dinitroxyde caractérisés par les propriétés discutées précédemment et par le fait qu'ils comportent également des groupements chimiques facilitant leur solubilité dans l'eau. Ces groupements facilitant la solubilité dans l'eau permettent d'utiliser les composés biradicalaires de la présente invention dans des milieux aqueux compatibles avec l'utilisation des composés de la présente invention milieux aqueux compatibles avec l'utilisation des composés de la présente invention dans de nombreuses applications biologiques. Les composès biradicalaires de la présente invention contiennent au moins un tel groupement chimique sélectionné, à titre d'exemples, parmi les fonctions et groupements suivants : hydroxyle (-OH), amine primaire (-NH₂), amine secondaire (-NH), tertiaire (-N), ammonio (-N⁺), oxyde d'amine (-NO), carboxyle (-COOH), sulfanyle (-SH), sulfinyle (-SO), sulfonyle (-SO₂), sulfonato (-SO₃), phosphono (-PO₃H₂, -PO₃R₂ où R= (C1-C17) alkyle, (C6-C18) aryle), phosphoryle (-P(O)R₂ où R= (C1-C17) alkyle, (C6-C18) aryle), phosphonio (-P⁺), ester phosporique (-O-PO₃⁻).

Dans un autre mode de réalisation, la prèsente invention concerne des composés biradicalaires de type dinitroxyde caractérisés par les propriétés discutées précédemment et par le fait qu'ils comportent également des groupements chimiques réactifs vis à vis de groupements caractéristiques -SH, -NH₂, -OH, -COOH et hydroxyaryle (ArOH). Ces groupements chimiques permettent la conjugaison des composés biradicalaires de type dimitroxyde de la presente invention à des molécules simples ou complexes à des fins analytiques. Par molecules simples auxquelles peuvent être conjugués les composés biradicalaires de la présente invention, on entend des molécules comportant au moins un groupe photosensibilisateur tel que, par exemple, la thioxanthone, la benzoïne, le benzil dimethylcétal ou la benzophénone. Par molécules complexes auxquelles peuvent être conjugués les composés biradicalaires de la présente invention, on entend principalement les biomolécules telles que protéines [de structure, récepteurs, de signalisation, de la machinerie cellulaire de transcription (polymérases), de la machinerie cellulaire de traduction (ribosomes)], lipides, saccharides, Acides Deoxyribonucléiques (ADN) et Acides Ribonucléiques (ARN). Quatre exemples de groupements chimiques permettant la conjugaison des composés biradicalaires de la présente invention sont présentés ci-dessous sans que cette liste ne soit exhaustive:

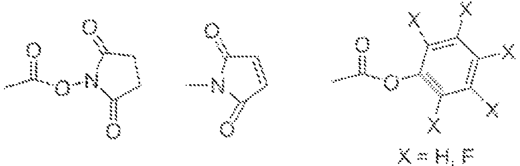

Par composés marqués isotopiquement, on comprend tous les dérivés des composés biradicalaires de type dinitroxyde concernés par le périmètre décrit précédémment dans la présente invention, obtenus par marquage radioisotopique aux différents éléments utiles dans les techniques de polarisation dynamique nucléaire, de biologie structurale, de Résonance Magnétique Nucléaire du solide ou appliquées à des échantillons liquides, de physique des particules et dans les techniques d'imagerie médicale. Les principaux éléments qui peuvent marquer les composés biradicalaires de la présente invention sont le Deutérium (²H), l'Azote 15 (¹⁵N) et le Carbone 13 (¹³C). sans que cette liste ne soit limitative.

Par composition, on comprend toute solution ou toute formulation injectable ou non, assimilable ou non, qui contient l'un des composés biradicalaires de type dinitroxyde de la présente invention sous forme pure ou en association avec un ou plusieurs autres produits, c'est à dire tout mélange comprenant un certain pourcentage d'au moins l'un des motifs biradicalaires de type dinitroxyde décrit dans la présente invention. Il est entendu que ces mélanges et compositions concernent les dérivés des composés biradicalaires dinitroxyde de la présente invention tels que les dérivés présentant un groupement chimique ajouté, les dérivés conjugués à une molécule d'intérêt on encore les dérivés polynitroxyde de ces composés. Des mélanges formés des composés et/ou dérivés de ces composés de l'invention et d'excipients sont aussi compris dans le périmètre de l'invention ainsi que les formes de ce mélange, liquides, solides, sous forme de gels ou autres.

Par utilisation on comprend toute utilisation qui peut être faite des composés compris dans le périmètre de l'invention. L'utilisation principale des composés de l'invention concerne leur utilisation comme agents de polarisation dans des techniques telles que la Polarisation Dynamique Nucléaire mais également dans des techniques telles que la Résonance Magnétique Nucléaire du solide ou appliquées à des échantillons liquides. L'utilisation des composés de la présente invention couvre les domaines de la chimie, de la biologie à travers les techniques de la biologie structurale mais également le domaine de la physique à travers les techniques de physique des particules. L'utilisation des composés de l'invention concerne le domaine médical, principalement celui de l'imagerie médicale telle que l'utilisation des composés de la présente invention en IRMN mais également celui du diagnostic.

### Example de préparation et d'utilisation en Polarisation Dynamique Nucléaire d'un composé biradicalaire de type dinitroxyde de la présente invention : cas du bTbK.

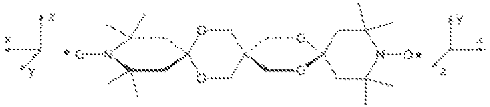

### Préparation de la diamine précurseur du bTbK

Un mélange de 2,2,6,6-tetraméthyl-4-pipéridone (2,00 g, 12,8 mmol), pentaerythritol (0,87 g, 6,4 mmol) et d'acide p-toluénesulfonique (2,21 g, 12,8 mmol) dans 50 ml de toluène est mis à reflux pendant 12 h, l'eau formée est éliminée grâce à un piège Dean-Stark. Après avoir refroidi le mélange, celui-ci est lavé avec une solution 1 N de NaOH (20 mL) puis 20 mL d'eau. La solution organique est séchée sur du Na₂SO₄ et le solvant est évaporé sous pression réduite. Une cristallisation dans un mélange toluéne/éther de pétrole (1 :1) fournit des cristaux incolores.
Rendement : 72%.
¹H RMN (300 MHz, CDCl₃) δ 3,76 (s, 8 H), 1,67 (s, 8 H), 1,20 (s, 24 H) ; ¹³C RMN (75 MHz, CDCl₃) δ 99,8 (2 C), 63,7 (4 C), 54,5 (4 C), 51,3 (4 C), 43,2 (1C) 32,4 (8 C). ESI-HRMS *m*/*z* [M+H]⁺; calc: 411,3217, trouve 411,3217.

### Préparation du bTbK

A une solution de la diamine (0,50 g, 1,2 mmol) dans 20 mL de CH₂Cl₂ on additionne à 0° C une solution de m-CPBA (1,5 eq., 1,8 mmol) dans 10 mL de CH₂Cl₂. Le mélange est agité pendant 1 h à température ambiante. Le mélange est lavé avec successivement une solution à 10 % de NaHCO₃ (10 mL), une solution (0,1 N) de HCl et 10 mL d'eau. La solution organique est séchée sur du Na2SO4 puis le solvant est évaporé sous pression réduite. Le bTbK est purifié par chromatographie sur une colonne de gel de silice avec un mélange CH₂Cl₂/EtOH (98 :2) comme éluant. Une recristallisation dans un mélange pentane/diethyl ether (50 :50) fournit des cristaux légèrement orange de bTbK.
Rendement 50 %.
ESI-HRMS *m*/*z* [M+H]⁺: trouvé: 441, calculé: 440.

### Analyse du bTbK par diffracton des rayons X

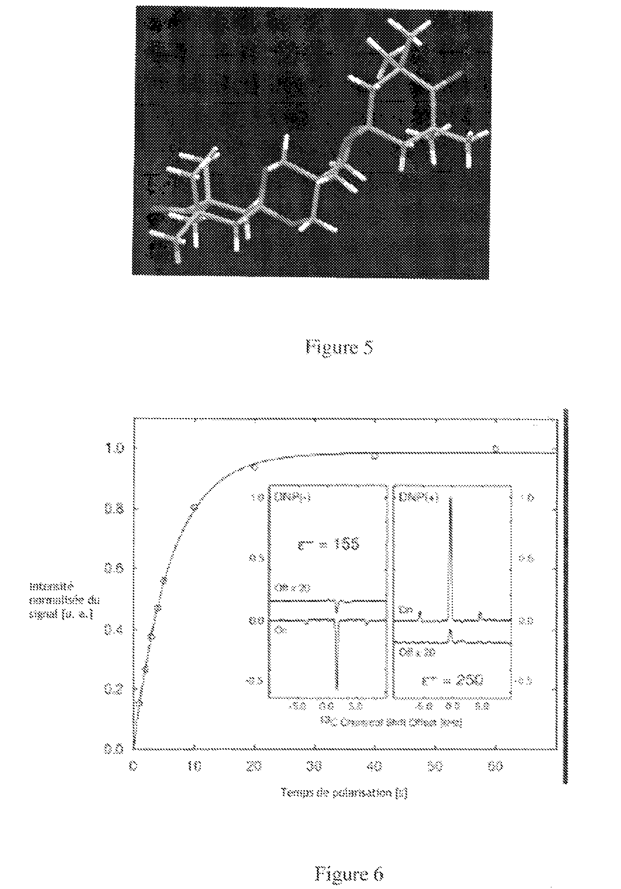

Une analyse par diffraction des rayons X a permis de determiner la géométrie exacte du bTbK (Figure 5). La géométrie du bTbK déterminée par diffraction des rayons X a fourni les données suivantes concernant la disposition des deux unités nitroxyde :
- D_{O-O} = 13 À, D_{N-M} = 10, 7 Å ce qui correspond à une distance moyenne de 11,8 Å entre les deux électrons célibataires des deux unités nitroxyde.
- L'angle que font entre eux les plans nodaux des groupes aminoxyle des deux unités nitroxyde est de 82°.

### Utilisation du bTbK comme agent de polarisation en PDN. Application à la polarisation du ¹³C de l'urée. Comparaison des performances du bTbK et du TOTAPOL.

Des experiences de PDN utilisant le bTbK comme agent de polarisation ont été effectuées dans un champ magnétique de 5 T, correspondant à une fréquence .de Larmor de 140 GHz pour e⁻ et 211 GHz pour ¹H. La polarisation des ¹H est détectée après son transfert au ¹³C de l'urée dont on enregistre le signal de RMN. L'expérience est conduite à 93 K dans un spectromètre PDN/RMN équipée d'une sonde MAS cryngénique à triple résonance (e⁻. ¹H et ¹³C). L'irradiation de l'échantillon est réalisée á l'aide d'un gyrotron capable de produire des ondes millimétriques de forte puissance (> 10 mW) à une fréquence de 139,662 GHz. L'aimant supraconducteur de 5 T est équipé d'une bobine de balayage supraconductrice qui permet de faire un balayage de champ de ± 750G, afin d'ajuster le champ magnétique pour observer la polarisation maximale. Les échantillons pour la DNP sout préparés à partir de solutions 9 mM en bTbk et 2M en urée marquée au ¹³C dans un mélange d₆-DMSO/D₂O/H₂O (77/17/6). La forte concentration en ¹³C-arée permet d'optimiser la détection RMN et d'observer le signal RMN dans un délai raisonnable en l'absence de polarisation.

Une courbe typique de croissance de la polarisation des ¹H du milieu est prèsentée sur la figure 6, ainsi que les spectres de RMN enregistrés avec ou sans irradiation à des champs correspondants aux maxima des polarisations positives DNP(+) et négatives DNP(-). La polarisation des ¹H du milieu est suivie en la transférant par une séquence de polarisation croisée au ¹³C de l'urée. A la température de 93 K, la constante de temps d'accroissement de la polarisation est de 7 s et l'état stationnaire atteint correspond à un facteur d'accroissement ε⁻ = 250 pour les polarisations positives, pour une puissance d'irradiation de l'ordre de 2,5 W.

Dans les mêmes conditions expérimentales, le TOTAPOL (H. G. Griffin et al : J. Am. Chem, Soc., 128, 13385-11390, 2006 ; J. Chem. Phys, 128, 053302-1/052302-17 2008) qui était jusqu'à ce jour le biradical du type dinitroxyde le plus performant dans ce type d'expérience présente un ε⁺ de 180.

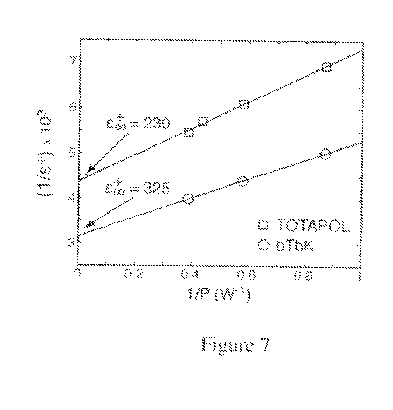

La diffèrence d'efficacité entre le bTbK et le TOTAPOL se traduit également dans la valeur des coefficients d'accroissement de la polarisation pour ces deux agents polarisants, lorsque la valeur de la puissance d'irradiation tend vers l'infini (Figure 7). Les valeurs trouvées pour 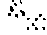 sont respectivement 230 et 325 pour le TOTAPOL et le bTbK.

Ces expériences démontrent la supériorité de bTbK par rapport aux autres biradicalaires du type di-TEMPO testés précédemment. Les facteurs d'accroissement de la polarisation mesurés avec le bTbK sont environ 1,4 fois plus grande que ceux mesurés avec le TOTAPOL malgré une moins bonne solubilité dans l'eau qui limite ses performances.

## Revendications

1. Composés biradicalaires du type dinitroxyde comme agent de polarisation pour la mise en oeuvre de techniques de Résonance Magnétique Nucléaire du solide ou appliquées à des échantillons liquides, dans les techniques de physique des particules ainsi que dans les techniques d'imagerie médicale, **caractérisés en ce qu'**ils présentent entre les deux unités nitroxyde une jonction rigide maintenant une orientation et une distance particulières entre les deux groupes aminoxyle de ces unités, ladite jonction rigide étant un nombre impair de liaisons spiraniques, et **en ce qu'**ils sont de formule générale : 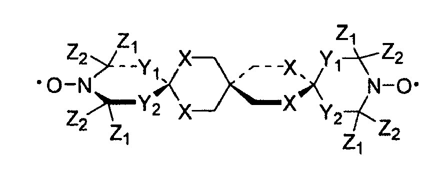 dans laquelle X est une simple liaison, ou O, N, N(O), S, S(O), SO₂, ou une chaîne (C1-C4) alkyle,
dans lesquelles Y1 est une simple liaison, ou O, N, N(O), S, S(O), SO₂, ou une chaîne (C1-C4) alkyle,
dans lesquelles Y2 est une simple liaison, ou O, N, N(O), S, S(O), SO₂, ou une chaîne (C1-C4) alkyle,
dans lesquelles Z₁ ou Z₂ = -R₁, et R2 est un éventuel substituant de R1, avec R₁ étant une chaîne (C1-C17) alkyle, (C1-C17) alkényle, (C1-C17) alkynyle, (C1-C17) cycloalkyle, (C1-C17) hétérocycloalkyle, (C6-C18) aryle et R₂ = H ou est choisi parmi un hydroxyle (-OH), amine primaire (-NH₂), amine secondaire(-NH), tertiaire(-N), ammonio (-N⁺), oxyde d'amine (-NO), carboxyle (-COOH), sulfanyle(-SH), sulfinyle (-SO), sulfonyle (-SO₂), sulfonato (-SO₃⁻), phosphono (-PO₃H₂, -PO₃(R)₂ où R= (C1-C17) alkyle, (C6-C18) aryle), phosphoryle (-P(O)(R)₂ où R= (C1-C17) alkyle, (C6-C18) aryle), phosphonio (-P⁺), ester phosphorique (-O-PO₃), et Z1 et Z2 combinés ensemble avec le même atome de carbone cyclique auquel ils sont liés peuvent former un (C1-C17) spirocycloalkyle ou un (C1-C17) spirohétérocycloalkyle éventuellement substitué par R2.

2. Composés biradicalaires de type dinitroxyde selon la revendication 1, **caractérisés en ce que** la jonction entre les deux unités nitroxyde permet l'obtention d'une conformation stable dans laquelle les plans nodaux des groupes aminoxyle de ces deux unités font entre eux un angle compris entre 65° et 115°.

3. Composés biradicalaires de type dinitroxyde selon la revendication 1, **caractérisés en ce que** la jonction entre les deux unités nitroxyde permet le maintien des atomes d'azote des groupes aminoxyle des unités nitroxyde à une distance comprise entre 3 et 16 Å.

4. Composés biradicalaires de type dinitroxyde selon la revendication 1, **caractérisés en ce que** les unités nitroxyde sont sélectionnées parmi les unités pipéridinoxyle, pyrrolidinoxyle, imidazolinoxyle, imidazolidinoxyle, oxazolidinoxyle et nitronylnitroxide.

5. Composés biradicalaires de type dinitroxyde selon les revendications 1 à 4, **caractérisés par** l'adjonction d'au moins un groupe photosensibilisateur sélectionné parmi la thioxanthone, la benzoïne, le benzyldiméthylcétal ou la benzophénone.

6. Composés biradicalaires de type dinitroxyde selon les revendications 1 à 5, **caractérisés par** l'adjonction d'au moins un groupement chimique facilitant la solubilité, sélectionné parmi les fonctions et les groupements hydroxyle (-OH), amine primaire (-NH₂), amine secondaire (-NH), tertiaire(-N), ammonio (-N⁺), oxyde d'amine (-NO), carboxyle (-COOH), sulfanyle (-SH),
sulfinyle (-SO), sulfonyle (-SO₂), sulfonato (-SO₃⁻), phosphono (-PO₃H₂, -PO₃ (R)₂ où R= (C1-C17) alkyle, (C6-C18) aryle), phosphoryle (-P(O)(R)₂ où R= (C1-C 17) alkyle, (C6-C 18) aryle), phosphonio (-P⁺), ester phosphorique (-O-PO₃⁻⁻).

7. Composés biradicalaires de type dinitroxyde selon les revendications 1 à 6, **caractérisés par** l'adjonction d'au moins un groupement chimique réactif vis à vis des groupements -SH, -NH₂, -NH-, -OH, -COOH, hydroxyaryle (-ArOH).

8. Composés biradicalaires de type dinitroxyde selon la revendication 7, **caractérisée en ce que** ledit au moins un groupement chimique réactif est sélectionné dans le groupe comprenant :

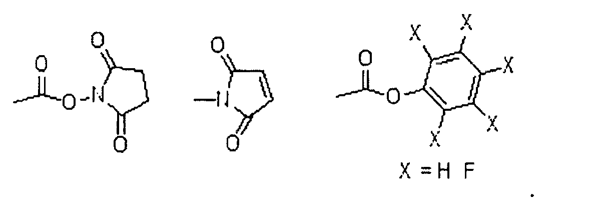

9. Composés biradicalaires de type dinitroxyde selon les revendications 1 à 8, **caractérisés par** leur conjugaison à des biomolécules sélectionnées parmi les protéines, ribosomes, lipides, saccharides, ADN, ARN.

10. Composés biradicalaires de type dinitroxyde selon les revendications 1 à 9, **caractérisés en ce qu'**ils sont marqués isotopiquement par un composé sélectionné parmi le Deutérium (²H), le Carbone 13 (¹³C) et l'Azote 15 (¹⁵N).

11. Composés biradicalaires de type dinitroxyde selon les revendications 1 à 10 comme agent de polarisation pour la mise en oeuvre des techniques de polarisation dynamique nucléaire.

12. Composé biradicalaires de type dinitroxyde selon la revendication 11 de formule générale :

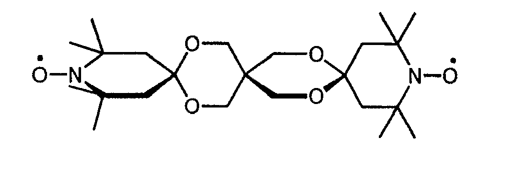

13. Composés paramagnétiques comprenant au moins un motif dinitroxyde composé par les deux unités nitroxyde reliées par une jonction rigide selon l'une des revendications 1 à 12, comme agent de polarisation pour la mise en oeuvre de techniques de Résonance Magnétique Nucléaire du solide ou appliquées à des échantillons liquides, dans les techniques de physique des particules ainsi que dans les techniques d'imagerie médicale.

14. Composition comprenant au moins un des composés biradicalaires de type dinitroxyde selon l'une des revendications 1 à 13.

## Patentansprüche

1. Dinitroxidbiradikalverbindungen als Polarisierungsagens zur Durchführung von Kernmagnetresonanz-Techniken für Festkörper oder flüssige Proben, in Techniken der Teilchenphysik wie auch in Techniken der Bildgebung in der Medizin, **dadurch gekennzeichnet, dass** sie zwischen den beiden Nitroxid-Einheiten eine starre Verknüpfung aufweisen, die eine bestimmte Ausrichtung und einen bestimmten Abstand zwischen den beiden Aminoxyl-Gruppen dieser Einheiten aufrechterhält, wobei besagte starre Verknüpfung eine ungerade Anzahl an Spirobindungen aufweist, und **dadurch gekennzeichnet, dass** sie die allgemeine Formel aufweisen: 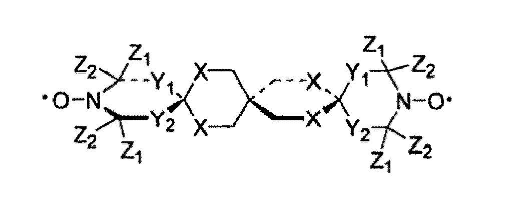 wobei X eine Einfachbindung oder O, N, N(O), S, S(O), SO₂ oder eine (C1-C4)-Alkylkette ist, wobei Y₁ eine Einfachbindung oder O, N, N(O), S, S(O), SO₂ oder eine (C1-C4)-Alkylkette ist, wobei Y₂ eine Einfachbindung oder O, N, N(O), S, S(O), SO₂ oder eine (C1-C4)-Alkylkette ist, wobei Z₁ oder Z₂ = -R₁ und R₂ ein eventueller Substituent von R₁ ist, wobei R₁ eine (C1-C17)-Alkylkette, (C1-C17)-Alkenylkette, (C1-C17)-Alkinylkette, ein (C1-C17)-Cycloalkyl, (C1-C17)-Heterocycloalkyl, (C6-C18)-Aryl ist und R₂ = H oder aus einem Hydroxyl (-OH), einem primären Amin (-NH₂), sekundären Amin (-NH), tertiären Amin (-N), Ammonium (-N⁺), Aminoxid (-NO), Carboxyl (-COOH), Sulfanyl (-SH), Sulfinyl (-SO), Sulfonyl (-SO₂), Sulfonat (-SO₃⁻), Phosphono (-PO₃H₂, -PO₃(R)₂, wo R = (C1-C17)-Alkyl, (C6-C18)-Aryl), Phosphoryl (-P(O)(R)₂, wo R = (C1-C17)-Alkyl, (C6-C18)-Aryl), Phosphonium (-P⁺), Phosphorester (-O-PO₃) ausgewählt ist, und Z₁ und Z₂ gemeinsam mit demselben cyclischen Kohlenstoffatom, an dem sie gebunden sind, ein eventuell mit R₂ substituiertes (C1-C17)-Spirocycloalkyl oder (C1-C17)-Spiroheterocycloalkyl bilden können.

2. Dinitroxidbiradikalverbindungen gemäß Anspruch 1, **dadurch gekennzeichnet, dass** die Verknüpfung zwischen den beiden Nitroxid-Einheiten den Erhalt einer stabilen Konformation erlaubt, in der die Knotenebenen der Aminoxylgruppen dieser beiden Einheiten miteinander einen Winkel zwischen 65° und 115° bilden.

3. Dinitroxidbiradikalverbindungen gemäß Anspruch 1, **dadurch gekennzeichnet, dass** die Verknüpfung zwischen den beiden Nitroxid-Einheiten die Einhaltung eines Abstandes zwischen den Stickstoffatomen der Aminoxylgruppen der Nitroxid-Einheiten zwischen 3 und 16 Å erlaubt.

4. Dinitroxidbiradikalverbindungen gemäß Anspruch 1, **dadurch gekennzeichnet, dass** die Nitroxid-Einheiten aus Piperidinoxyl-, Pyrrolidinoxyl-, Imidazolinoxyl-, Imidazolidinoxyl-, Oxazolidinoxyl- und Nitronylnitroxid-Einheiten ausgewählt sind.

5. Dinitroxidbiradikalverbindungen gemäß Ansprüchen 1 bis 4, die durch Anfügen wenigstens einer lichtempfindlichen Gruppe gekennzeichnet sind, die aus Thioxanthon, Benzoin, Benzyldimethylketal oder Benzophenon ausgewählt ist.

6. Dinitroxidbiradikalverbindungen gemäß Ansprüchen 1 bis 5, die durch Anfügen wenigstens einer die Löslichkeit fördernden chemischen Gruppe gekennzeichnet sind, die aus den Funktionen und den Gruppen wie einem Hydroxyl (-OH), primären Amin (-NH₂), sekundären Amin (-NH), tertiären Amin (-N), Ammonium (-N⁺), Aminoxid (-NO), Carboxyl (-COOH), Sulfanyl (-SH), Sulfinyl (-SO), Sulfonyl (-SO₂), Sulfonat (-SO₃⁻), Phosphono (-PO₃H₂, -PO₃(R)₂, wo R = (C1-C17)-Alkyl, (C6-C18)-Aryl), Phosphoryl (-P(O)(R)₂, wo R = (C1-C17)-Alkyl, (C6-C18)-Aryl), Phosphonium (-P⁺), Phosphorester (-O-PO₃) ausgewählt ist.

7. Dinitroxidbiradikalverbindungen gemäß Ansprüchen 1 bis 6, die durch Anfügen wenigstens einer Gruppe gekennzeichnet sind, die mit den Gruppen -SH, -NH₂, -NH-, -OH, COOH, Hydroxylaryl (-ArOH) chemisch reaktiv ist.

8. Dinitroxidbiradikalverbindungen gemäß Anspruch 7, **dadurch gekennzeichnet, dass** besagte wenigstens eine chemisch reaktive Gruppe aus der Gruppe ausgewählt ist, umfassend:

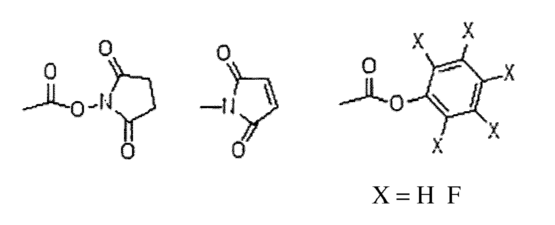

9. Dinitroxidbiradikalverbindungen gemäß Ansprüchen 1 bis 8, die durch ihre Konjugation an Biomoleküle gekennzeichnet sind, die aus Proteinen, Ribosomen, Lipiden, Sacchariden, DNA, RNA ausgewählt sind.

10. Dinitroxidbiradikalverbindungen gemäß Ansprüchen 1 bis 9, **dadurch gekennzeichnet, dass** sie durch eine Verbindung mit Isotopen markiert sind, die aus Deuterium (²H), Kohlenstoff 13 (¹³C) und Stickstoff 15 (¹⁵N) ausgewählt sind.

11. Dinitroxidbiradikalverbindungen gemäß Ansprüchen 1 bis 10 als Polarisierungsagens zur Durchführung von dynamischen Kernpolarisierungstechniken.

12. Dinitroxidbiradikalverbindungen gemäß Anspruch 11 mit der allgemeinen Formel:

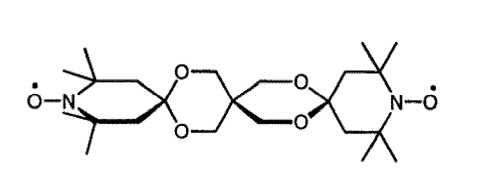

13. Paramagnetische Verbindungen, umfassend wenigstens eine Dinitroxid-Einheit, die aus zwei Nitroxid-Einheiten besteht, die durch eine starre Verknüpfung miteinander verbunden sind, gemäß Ansprüchen 1 bis 12 als Polarisierungsagens zur Durchführung von Kernmagnetresonanz-Techniken für Festkörper oder flüssige Proben, in Techniken der Teilchenphysik wie auch in Techniken der Bildgebung in der Medizin.

14. Zusammensetzung, umfassend wenigstens eine der Dinitroxidbiradikalverbindungen gemäß einem der Ansprüche 1 bis 13.

## Claims

1. A biradical compounds of dinitroxide type as polarizing agent for the implementation of solid-state or liquid-state Nuclear Magnetic Resonance techniques, of particle physics techniques and of medical imaging techniques, are **characterized in that** between the two nitroxide units they have a rigid junction maintaining a particular orientation and distance between the two aminoxyl groups of these units, the said rigid junction being an uneven number of spiranic bonds, and **in that** they have the general formula: 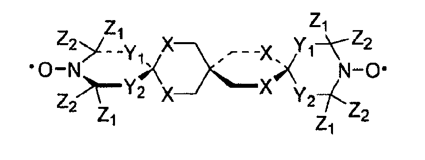 where X is a simple bond, or O, N, N(O), S, S(O), SO₂, or a (C1-C4) alkyl chain, where Y1 is a simple bond, or O, N, N(O), S, S(O), SO₂ or a (C1-C4) alkyl chain, where Y2 is a simple bond or O, N, N(O), S, S(O), SO₂ or a (C1-C4) alkyl chain, where Z₁ or Z₂ = -R₁, and R₂ is an optional substituent of R₁, with R₁ being a (C1-C17) alkyl, (C1-C17) alkenyl, (C1-C17) alkynyl, (C1-C17) cycloalkyl, (C1-C17) heterocycloalkyl, (C6-C18) aryl chain, and R₂= H or is chosen from among a hydroxyl (-OH), primary amine (-NH₂), secondary amine (-NH), tertiary (-N), ammonio (-N⁺), amine oxide (-NO), carboxyl (-COOH), sulfanyl (-SH), sulfinyl (-SO), sulfonyl (-SO₂), sulfonato (-SO₃-), phosphono (-PO₃H₂, -PO₃(R)₂ where R= (C1-C17) alkyl, (C6-C18) aryl), phosphoryl (-P(O)(R)₂ where R =(C1-C17) alkyl, (C6-C18) aryl), phosphonio (-P⁺), phosphoric ester (-O-PO₃), and Z1 and Z2 together combined with the same cyclic carbon atom to which they are bonded may form a (C1-C17) spirocycloalkyl or (C1-C17) spiroheterocycloalkyl optionally substituted by R₂.

2. The biradical compounds of dinitroxide type according to claim 1, **characterized in that** the junction between the two nitroxide units allows a stable conformation to be obtained wherein the nodal planes of the aminoxyl groups of these two units form an angle between them of between 65° and 115°.

3. The biradical compounds of dinitroxide type according to claim 1, **characterized in that** the junction between the two nitroxide units allows the maintaining of the nitrogen atoms of the aminoxyl groups of the nitroxide units at a distance of between 3 and 16 Å.

4. The biradical compounds of dinitroxide type according to claim 1, **characterized in that** the nitroxide units are chosen from among piperidinoxyl, pyrrolidinoxyl, imidazolinoxyl, imidazolidinoxyl, oxazolidinoxyl and nitronylnitroxide units.

5. The biradical compounds of dinitroxide type according to claims 1 to 4, **characterized by** the addition of at least one photosensitizing group chosen from among thioxanthone, benzoin, benzyl dimethyl ketal or benzophenone.

6. The biradical compounds of dinitroxide type according to claims 1 to 5, **characterized by** the addition of at least one chemical group facilitating solubility chosen from among functions and groups: hydroxyl (-OH), primary amine (-NH₂), secondary amine (-NH), tertiary (-N), ammonio (-N⁺), amine oxide (-NO), carboxyl (-COOH), sulfanyl (-SH), sulfinyl (-SO), sulfonyl (-SO₂), sulfonato (-SO₃⁻) phosphono (-PO₃H₂, -PO₃(R)₂, where R = (C1-C17) alkyl, (C6-C18) aryl), phosphoryl (-P(O)(R)₂ where R = (C1-C17) alkyl, (C6-C18) aryl), phosphonio (-P⁺), phosphoric ester (-O-PO₃⁻⁻).

7. The biradical compounds of dinitroxide type according to claims 1 to 6, **characterized by** the addition of at least one chemical group reactive against -SH, -NH₂, -NH-, -OH, -COOH, hydroxyaryl (-ArOH) groups.

8. The biradical compounds of dinitroxide type according to claim 7, **characterized in that** the said at least one reactive chemical group is chosen from the group comprising:

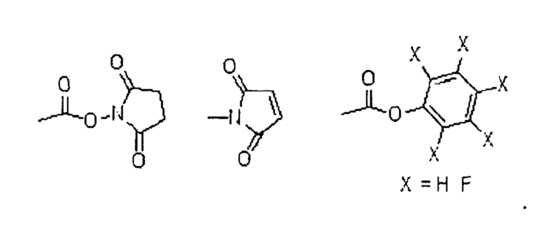

9. The biradical compounds of dinitroxide type according to claims 1 to 8, **characterized by** their conjugation with biomolecules chosen from among proteins, ribosomes, lipids, saccharides, DNA, RNA.

10. The biradical compounds of dinitroxide type according to claims 1 to 9 **characterized in that** they are isotopically labelled by a compound chosen from among Deuterium (²H), Carbon 13 (¹³C) and Nitrogen 15 (¹⁵N).

11. The biradical compounds of dinitroxide type according to claims 1 to 10 as polarizing agent for implementing dynamic nuclear polarizing techniques.

12. The biradical compound of dinitroxide type according to claim 11 of general formula:

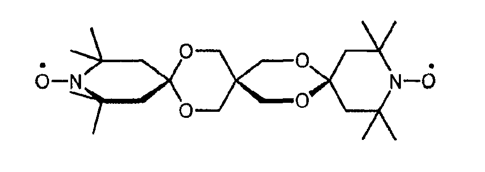

13. A paramagnetic compounds comprising at least one dinitroxide repeat unit formed by the two nitroxide units linked by a rigid junction according to one of claims 1 to 12, as polarizing agent for implementing solid-state or liquid-state Nuclear Magnetic Resonance techniques, particle physics techniques and medical imaging techniques.

14. A composition comprising at least one of the biradical compounds of dinitroxide type according to one of claims 1 to 13.
